# EUROPEAN PATENT APPLICATION

(11) **EP 4 494 622 A1**
(43) Date of publication of application: **22.01.2025**
(21) Application number: 23826999.7
(22) Date of filing: 08.06.2023
(51) Int. Cl.: A61F 13/49, A61F 13/496, A61F 13/535, A61F 13/84

(54) **ABSORBENT ARTICLE**

(30) Priority: 20.06.2022 JP 2022099152
(71) Applicant: Unicharm Corporation, Ehime 799-0111 (JP)
(72) Inventor: OHTSUBO, Toshifumi, Kanonji-shi, Kagawa 769-1602 (JP); KANEKO, Tomohiro, Kanonji-shi, Kagawa 769-1602 (JP); NAGATOMO, Shoki, Kanonji-shi, Kagawa 769-1602 (JP)
(74) Representative: Dolleymores
(86) International application number: PCT/JP2023/021341
(87) International publication number: WO 2023/248812

(57) **Abstract**

Provided is an absorbent article comprising a stretchable region having elastic members (23, 33) that are stretchable in the left-right direction and are disposed in at least one of an abdominal torso part (20) and a dorsal torso part (30). The value obtained by dividing a first measurement value by a second measurement value is less than 1.2, where the first measurement value is a value obtained by using at least a portion of the stretchable region as a test piece and dividing, by the width of the test piece in the up-down direction, a force measured when the test piece is elongated in the left-right direction at a rate of 50000 mm/min to a length of 84% of the length thereof at maximum elongation, and the second measurement value is a value obtained by dividing, by said width, a force measured when the test piece is elongated in the left-right direction at a rate of 500 mm/min to a length of 84% of the length thereof at maximum elongation.

## Description

### FIELD

The present invention relates to an absorbent article.

### BACKGROUND

Patent Literature 1 discloses a stretchable sheet in which, when Stretched by a tensile tester to twice the original length at 300 mm/min and then allowed to contract to obtain the relationship between stress and the stretch ratio, the value of B/A is 0.83 or more and 0.90 or less, where A is the stress when stretched to 1.8 times the original length in the second cycle, and B is the stress when allowed to contract to 1.8 times the original length in the second cycle. It is described that when this stretchable sheet is used in the exterior body of an absorbent article, A is an indicator of how easily the absorbent article can be spread open, B is an indicator of how well the absorbent article fits to the wearer, and the closer the value of B/A is to 1, the easier it is to move while wearing the absorbent article, and the less excessively tight the absorbent article feels while being worn.

### [CITATION LIST]

### [PATENT LITERATURE]

[Patent Literature 1] JP 2020-199248A

### SUMMARY

### [TECHNICAL PROBLEM]

However, when a user actually spreads open the exterior body (waist portion) of the absorbent article to put on the absorbent article, the absorbent article is stretched at a speed much faster than the stretching speed described in Patent Literature 1 (300 mm/min). Therefore, there is a risk that the value of A described above will deviate significantly from how easily the absorbent article can be spread open when putting on. On the other hand, while the absorbent article is being worn, the exterior body is stretched relatively slowly during wearer movement such as breathing. Therefore, the feeling of tightness felt by the wearer is likely to be influenced by not only the stress B during contraction as in Patent Literature 1, but also stretching stress when the exterior body is stretched slowly. In absorbent articles, there is a large difference between how easily the waist portion can spread open (stretch) when putting on and how easily the waist portion can spread open (stretch) while being worn, and the user may feel it difficult to spread open the waist portion when putting on the absorbent article. In this case, the user may be given the impression that the product is tight rather than the impression of the fit of the product while being worn.

The present invention has been made in consideration of the above-mentioned problems, and an aspect of the present invention is to reduce the difference in the ease of stretching of the waist portion of an absorbent article between when putting on the absorbent article and while the absorbent article is being worn, to suppress giving the user the impression that the absorbent article is an excessively tight product.

### [SOLUTION TO PROBLEM]

A main aspect of the present invention for achieving the aforementioned object is an absorbent article having a vertical direction and a lateral direction that intersect each other, the absorbent article including: an absorbent core; a front waist portion; and a back waist portion, at least either of the front waist portion and the back waist portion having a stretchable region in which an elastic member capable of stretching and contracting in the lateral direction is arranged, and in a test performed on a test piece that is at least a portion of the stretchable region, a value obtained by dividing a first measurement value by a second measurement value being smaller than 1.2, the first measurement value being a value obtained when a force measured when the test piece is stretched in the lateral direction to 84% of a maximum stretched length at a speed of 50000 mm/min is divided by a width of the test piece in the vertical direction, and the second measurement value being a value obtained when a force measured when the test piece is stretched in the lateral direction to 84% of the maximum stretched length at a speed of 500 mm/min is divided by the width of the test piece.

Other features of the present invention will become apparent from the following detailed description of the present invention and the accompanying drawings.

### [ADVANTAGEOUS EFFECT OF THE INVENTION]

According to the present invention, it is possible to reduce the difference in the ease of stretching of the waist portion of an absorbent article between when putting on the absorbent article and while the absorbent article is being worn, to suppress giving the user the impression that the absorbent article is an excessively tight product.

### BRIEF DESCRIPTION OF DRAWINGS

FIG. 1 is a schematic perspective view of an underpants-shaped disposable diaper 1.
FIG. 2 is a schematic plan view of the diaper 1 in an unfolded and stretched state, as viewed from the wearer's skin side.
FIG. 3 is a schematic cross-sectional view taken along line A-A in FIG. 2.
FIG. 4 is an illustrative diagram of weld portion rows Ra, Rb, and Rc provided in a front waist portion 20.
FIGS. 5A and 5B are illustrative diagrams of attachment of waist elastic members 23.
FIGS. 6A to 6C are enlarged views of portions of the weld portion rows Ra, Rb, and Rc.
FIGS. 7A and 7B are illustrative diagrams of a tensile test performed on waist portions 20 and 30.
FIG. 8 shows results of the tensile test.
FIG. 9 shows results of the tensile test.
FIGS. 10A and 10B are illustrative diagrams of the waist elastic member 23.

### DESCRIPTION OF EMBODIMENTS

At least the following matter will become apparent from the description of the present specification and the accompanying drawings.

An aspect 1 is an absorbent article having a vertical direction and a lateral direction that intersect each other, the absorbent article including: an absorbent core; a front waist portion; and a back waist portion, at least either of the front waist portion and the back waist portion having a stretchable region in which an elastic member capable of stretching and contracting in the lateral direction is arranged, and in a test performed on a test piece that is at least a portion of the stretchable region, a value obtained by dividing a first measurement value by a second measurement value being smaller than 1.2, the first measurement value being a value obtained when a force measured when the test piece is stretched in the lateral direction to 84% of a maximum stretched length at a speed of 50000 mm/min is divided by a width of the test piece in the vertical direction, and the second measurement value being a value obtained when a force measured when the test piece is stretched in the lateral direction to 84% of the maximum stretched length at a speed of 500 mm/min is divided by the width of the test piece.

According to the aspect 1, compared to when the value obtained by dividing the first measurement value by the second measurement value is 1.2 or more, the first measurement value measured during high-speed stretching can be suppressed, and the difference from the second measurement value measured during low-speed stretching can be reduced. Therefore, it is easier to spread (stretch) the waist portion when putting on the absorbent article, and it is possible to reduce the difference from the fit (tightness) while being worn. This makes it possible to suppress giving the user the impression that the absorbent article is a product that is too tight.

An aspect 2 is the absorbent article according to the aspect 1, wherein in a test in which the test piece is stretched to 84% of the maximum stretched length at a speed of 50000 mm/min and then, 15 seconds after a start of stretching, is allowed to contract to 66% of the maximum stretched length, a value obtained by dividing a fourth measurement value by a third measurement value is 0.95 or more, the third measurement value being a value obtained when a force measured when the test piece contracted to 66% of the maximum stretched length is divided by the width, and the fourth measurement value being a value obtained when a force measured 585 seconds after the test piece contracted to 66% of the maximum stretched length is divided by the width.

According to the aspect 2, compared to when the value obtained by dividing the fourth measurement value by the third measurement value is less than 0.95, it is possible to reduce the third measurement value measured when the waist portion is stretched a large amount and then allowed to contract, and to reduce the difference from the fourth measurement value measured when the contraction has stabilized. This makes it possible to reduce the feeling of tightness when the waist portion contracts to fit to the wearer when putting on the absorbent article (i.e., immediately after putting on the absorbent article). Conversely, even if the stretched state of the absorbent article continues in order to fit the wearer, the fit is maintained.

An aspect 3 is the absorbent article according to the aspect 1 or 2, wherein the absorbent article is an underpants-shape absorbent article in which the front waist portion and the back waist portion are joined to each other by a pair of side joining portions at two lateral side portions, and the test piece is a section that is in the front waist portion and the back waist portion joined in a ring-like shape by the side joining portions and that is located above a center, in the vertical direction, of the side joining portions.

According to the aspect 3, the upper half section of the waist portion is likely to influence the ease of spreading of the waist portion and the feeling of tightness when putting on the absorbent article. Therefore, in the upper half of the waist portion, if the value obtained by dividing the first measurement value by the second measurement value is 1.2 or less, or the value obtained by dividing the fourth measurement value by the third measurement value is 0.95 or more, the waist portion can be spread more easily, and the feeling of tightness immediately after putting on the absorbent article can be further reduced.

An aspect 4 is the absorbent article according to any one of the aspects 1 to 3, wherein in the stretchable region, the elastic member is arranged between a pair of sheets joined together by a plurality of weld portions, and while the elastic member is contracted in the lateral direction, a position, in the vertical direction, of the elastic member relative to the pair of sheets is restricted by the elastic member being sandwiched between two of the weld portions adjacent in the vertical direction.

According to the aspect 4, the amount of adhesive used in the waist portion can be reduced. Therefore, it is possible to reduce the influence of the hardness of the adhesive when the waist portion is stretched at a high speed, and it is possible to reduce the first measurement value and reduce the difference from the second measurement value.
the absorbent article is an underpants-shape absorbent article in which the front waist portion and the back waist portion are joined to each other by a pair of side joining portions at two lateral side portions, and in at least either of the front waist portion and the back waist portion, a plurality of the elastic members are arranged at intervals in the vertical direction, and for all of the elastic members that are overlapped with the side joining portions with respect to the vertical direction, while contracted in the lateral direction, positions, in the vertical direction, of the overlapping elastic members relative to the pair of sheets are restricted by the overlapping elastic members being sandwiched between vertically adjacent two of the weld portions.

According to the aspect 5, the amount of adhesive used in the waist portion can be reduced. Therefore, it is possible to reduce the influence of the hardness of the adhesive when the waist portion is stretched at a high speed, and it is possible to reduce the first measurement value and reduce the difference from the second measurement value.

An aspect 6 is the absorbent article according to the aspect 4 or 5, wherein the plurality of weld portions include a plurality of weld portion pairs each including two weld portions that are adjacent in the vertical direction and sandwich a corresponding elastic member, the plurality of weld portion pairs include a first weld portion pair, and a second weld portion pair located closer to a center in the lateral direction than the first weld portion pair is, and in the second weld portion pair, a distance in the vertical direction between the weld portions sandwiching the corresponding elastic member is larger than in the first weld portion pair.

According to the aspect 6, it is possible to suppress the case where the elastic member comes out of the first weld portion pair, and to ensure stretchability of the waist portion over a wide range in the lateral direction. The second weld portion pair can restrict the vertical position of the elastic member and also suppress rising of the sheets. Therefore, compared to the case where an adhesive is used to suppress rising of the sheets, the amount of adhesive used in the waist portion can be reduced, and it is possible to reduce the first measurement value and reduce the difference from the second measurement value.

An aspect 7 is the absorbent article according to any one of the aspects 4 to 6, wherein the plurality of weld portions include a plurality of weld portion pairs each including two weld portions that are adjacent in the vertical direction and sandwich a corresponding elastic member, the plurality of weld portion pairs include a third weld portion pair, and a fourth weld portion pair located closer to a center in the lateral direction than the third weld portion pair is, and in the fourth weld portion pair, lengths of the weld portions in the lateral direction are smaller than in the third weld portion pair.

According to the aspect 7, it is possible to suppress the case where the elastic member comes out of the third weld portion pair, and to ensure stretchability of the waist portion over a wide range in the lateral direction. The fourth weld portion pair can restrict the position of the elastic member and also reduce frictional force between the elastic member and the weld portions, and the waist portion can be spread more easily. This makes it possible to suppress giving the user the impression that the product is too tight.

An aspect 8 is the absorbent article according to any one of the aspects 4 to 7, wherein the stretchable region includes a first elastic member and a second elastic member adjacent to each other with an interval therebetween in the vertical direction, the plurality of weld portions include a first weld portion adjacent to the first elastic member, and a second weld portion located in a central portion, in the vertical direction, between the first elastic member and the second elastic member, and the first weld portion has a higher weld strength than the second weld portion.

According to the aspect 8, the position of the elastic member can be reliably restricted by the stiffer first weld portion. The less stiff second weld portion can ensure the flexibility of the waist portion and also suppress rising of the sheets. Therefore, compared to the case where an adhesive is used to suppress rising of the sheets, the amount of adhesive used in the waist portion can be reduced, and it is possible to reduce the first measurement value and reduce the difference from the second measurement value.

An aspect 9 is the absorbent article according to any one of the aspects 4 to 8, wherein while the stretchable region is stretched in the lateral direction, a length in the vertical direction of a gap between the two weld portions that are adjacent in the vertical direction and sandwich the elastic member is greater than a length in the vertical direction of the elastic member.

According to the aspect 9, as the waist portion contracts in the lateral direction, the elastic member becomes thinner and is less constrained by the weld portions. Therefore, the waist portion can be spread easily, and it is possible to suppress giving the user the impression that the product is too tight.

An aspect 10 is the absorbent article according to any one of the aspects 1 to 9, wherein the absorbent article is an underpants-shape absorbent article in which the front waist portion and the back waist portion are joined to each other by a pair of side joining portions at two lateral side portions, the absorbent article further comprises: an absorbent main body including the absorbent core; and an exterior body, and while the absorbent article is in an unfolded state, the stretchable region has a region in which a number of sheet layers constituting the exterior body is two layers corresponding to a pair of sheets sandwiching the elastic member.

According to the aspect 10, it is possible to reduce the size of the region where sheet layers are stacked and joined with an adhesive, and the amount of adhesive used in the waist portion can be reduced. Therefore, the first measurement value can be reduced, and the difference from the second measurement value can be reduced.

An aspect 11 is the absorbent article according to any one of the aspects 1 to 10, wherein the absorbent article is an underpants-shape absorbent article in which the front waist portion and the back waist portion are joined to each other by a pair of side joining portions at two lateral side portions, the absorbent article further comprises an absorbent main body including the absorbent core, and at least either of the front waist portion and the back waist portion includes a folded-back portion in which an upper end portion of at least either of a pair of sheets sandwiching the elastic member is folded downward in the vertical direction, and a region with no adhesive, between an upper end of the absorbent main body and a lower end of the folded-back portion.

According to the aspect 11, the amount of adhesive used in the waist portion can be reduced. Therefore, the first measurement value can be reduced, and the difference from the second measurement value can be reduced.

An aspect 12 is the absorbent article according to any one of the aspects 1 to 11, wherein a lubricant is applied to at least either of a surface of the elastic member and inward surfaces of the pair of sheets sandwiching the elastic member.

According to the aspect 12, the frictional force generated between the elastic member and the sheets can be reduced, and the waist portion can be spread easily. This makes it possible to suppress giving the user the impression that the product is too tight.

An aspect 13 is the absorbent article according to any one of the aspects 1 to 12, wherein the value obtained by dividing the first measurement value by the second measurement value when the back waist portion is the test piece is smaller than the value obtained by dividing the first measurement value by the second measurement value when the front waist portion is the test piece.

According to the aspect 13, the back waist portion can easily stretch even at a high speed, and the back waist portion can be easily spread wide to cover the buttocks when putting on the absorbent article.

An aspect 14 is the absorbent article according to any one of the aspects 1 to 12, wherein the value obtained by dividing the first measurement value by the second measurement value when the front waist portion is the test piece is smaller than the value obtained by dividing the first measurement value by the second measurement value when the back waist portion is the test piece.

According to the aspect 14, the front waist portion can stretch easily even at a high speed, and the front waist portion can be spread easily when putting on the absorbent article, thereby making it possible to suppress the case where the wearer's feet (particularly the wearer's toes) get caught when passing the wearer's feet through the waist opening.

An aspect 15 is the absorbent article according to any one of the aspects 1 to 13, wherein the absorbent article is an underpants-shape absorbent article in which the front waist portion and the back waist portion are joined to each other by a pair of side joining portions at two lateral side portions, and while the absorbent article is in an unfolded state, in a section of the absorbent core overlapped with the back waist portion in a thickness direction of the back waist portion, an average basis weight of the absorbent core is lower than in a section of the absorbent core overlapped with the front waist portion in a thickness direction of the front waist portion.

According to the aspect 13, the stiffness of the absorbent core is reduced in the back waist portion, and the back waist portion can be spread more easily. Therefore, the back waist portion can be easily spread wide to cover the buttocks of the wearer. On the other hand, by increasing the basis weight of the absorbent core in the front waist portion close to the urination area, it is possible to suppress leakage.

### Embodiments

Hereinafter, an underpants-shaped disposable diaper for infants will be described as an example of an absorbent article according to the present embodiment. However, the absorbent article is not limited to this, and may be any absorbent article having a front waist portion and a back waist portion, such as an underpants-shaped diaper for adults, a shorts-type sanitary napkin, or a tape-type diaper.

### Basic configuration of underpants-shaped disposable diaper 1

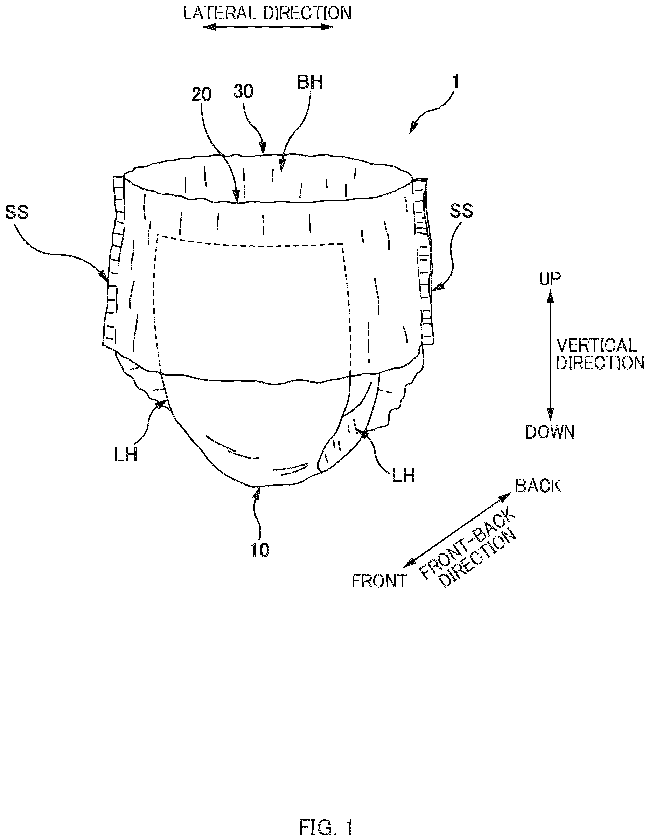

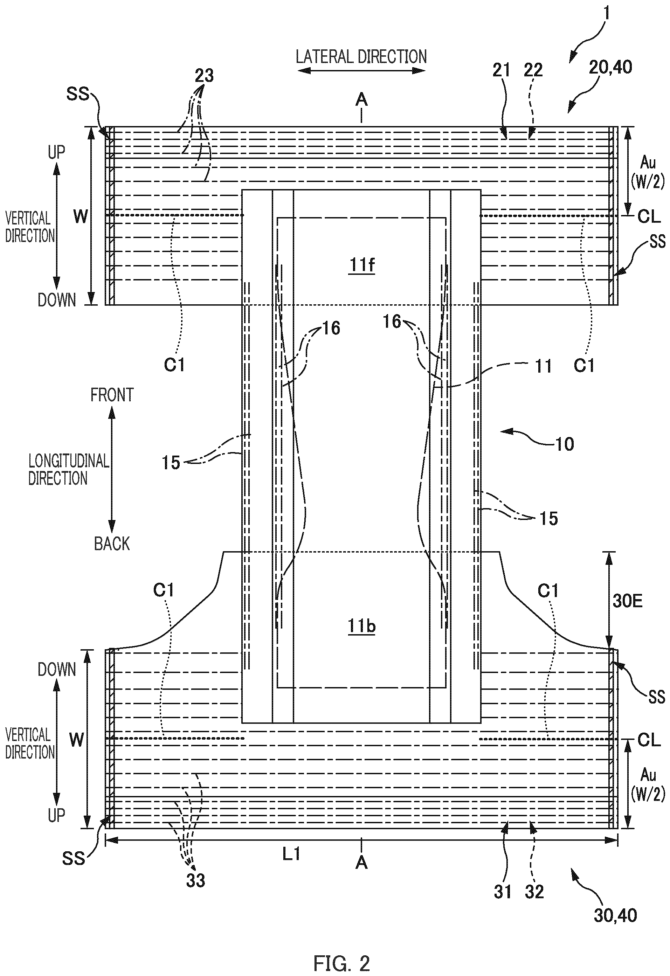

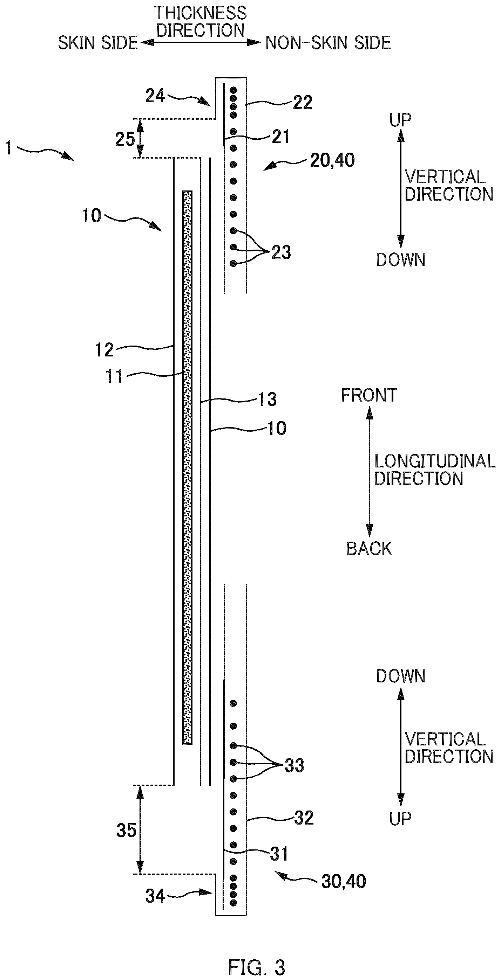

FIG. 1 is a schematic perspective view of an underpants-shaped disposable diaper 1 (hereinafter also referred to as "diaper"). FIG. 2 is a schematic plan view of the diaper 1 in an unfolded and stretched state, as viewed from the wearer's skin side. FIG. 3 is a schematic cross-sectional view taken along line A-A in FIG. 2.

The diaper 1 has a vertical direction, a lateral direction, and a front-back direction that intersect each other, and has a waist opening BH and a pair of leg openings LH. In the vertical direction, the side corresponding to the waist opening BH is the upper side, and the side corresponding to the wearer's crotch is the lower side. In the front-back direction, the side corresponding to the wearer's stomach is the front side, and the side corresponding to the wearer's back is the back side. Also, as shown in FIG. 3, the diaper 1 has a thickness direction in which members are stacked, and in the thickness direction, the side that comes into contact with the wearer is the skin side, and the opposite side is the non-skin side.

The diaper 1 includes an absorbent main body 10 and a pair of waist portions 20 and 30. The pair of waist portions 20 and 30 are joined to the non-skin-side surface of the absorbent main body 10 with an adhesive or the like. Out of the pair of waist portions 20, the one located on the front side will also be referred to as the front waist portion 20, and the one located on the back side will also be referred to as the back waist portion 30. The front waist portion 20 and the back waist portion 30 are joined to each other by a pair of side joining portions SS at side portions in the lateral direction. Examples of the joining by the side joining portions SS include heat welding, ultrasonic welding, and use of an adhesive.

As shown in FIG. 3, the absorbent main body 10 includes an absorbent core 11, a liquid-permeable top sheet 12 arranged on the skin side with respect to the absorbent core 11, a liquidimpermeable back sheet 13 arranged on the non-skin side with respect to the absorbent core 11, and an exterior sheet 14. The absorbent core 11 need to be any member that absorbs and retains excreted fluid, and examples thereof include those formed by molding a liquid-absorbent material such as pulp fibers or a superabsorbent polymer into a predetermined shape. The absorbent core 11 may also be covered with a liquid-permeable sheet made of tissue paper, nonwoven fabric, or the like.

As shown in FIG. 2, leg elastic members 15 (e.g., elastic strings) that stretch and contract along the longitudinal direction of the absorbent main body 10 may be provided in two lateral side portions of the absorbent main body 10. Furthermore, leak-proof wall portions capable of standing up toward the wearer's skin may be provided laterally inward of the leg elastic members 15. The leak-proof wall portions are formed by, for example, folding two lateral side portions of the exterior sheet 14 inward in the lateral direction so as to be located on the skin-side surface of the top sheet 12, and providing leak-proof-wall elastic members 16 (e.g., elastic strings) that stretch and contract in the longitudinal direction in the folded portions.

In the present embodiment, the waist portions 20 and 30 are portions that overlap the side joining portions SS with respect to the vertical direction, and the front waist portion 20 and the back waist portion 30 are portions that are rectangular in a plan view. An extension portion 30E having a substantially trapezoidal shape is provided on the crotch side of the back waist portion 30. The extension portion 30E can cover the buttocks of the wearer. In the following description, the back waist portion 30 and the extension portion 30E will be collectively referred to as the back exterior portions 30 and 30E. However, there is no limitation to this, and for example, the extension portion 30E need not be provided on the back side, or a portion extending from the front waist portion 20 toward the crotch may be provided.

The front waist portion 20 and the back exterior portions 30 and 30E respectively include skin-side sheets 21 and 31, non-skin-side sheets 22 and 32, and waist elastic members 23 and 33 (e.g., elastic strings) that can stretch and contract in the lateral direction. The waist elastic members 23 (33) are arranged between the skin-side sheet 21 (31) and the non-skin-side sheet 22 (32) at intervals in the vertical direction.

It is preferable that the skin-side sheets 21 and 31 and the non-skin-side sheets 22 and 32 are made of flexible sheet members, examples of which include spunbond nonwoven fabric, meltblown nonwoven fabric, and SMS (spunbond/meltblown/spunbond) nonwoven fabric. The constituent fibers of the nonwoven fabric are not limited to single fibers made of polypropylene (PP), a typical example of a thermoplastic resin, and may be single fibers made of other thermoplastic resins such as polyethylene (PE), or further may be composite fibers having a sheath-core structure made of PE and PP or the like.

Although the basic configuration of the diaper 1 has been described above, the above-described configuration of the diaper 1 is merely an example, and the present invention is not limited to this. For example, in the diaper 1 of the present embodiment, the front waist portion 20 and the back exterior portions 30 and 30E are formed from separate members, and the non-skin-side surface of the absorbent main body 10 is exposed in the crotch portion. However, there is no limitation to this, and, for example, the diaper may include an exterior member constituted by three members including the front waist portion 20, the back exterior portions 30 and 30E, and a crotch portion connecting them to each other, or the diaper may include an exterior member constituted by a single continuous member extending from the front waist portion 20 to the back waist portion 30.

### Waist portions 20 and 30

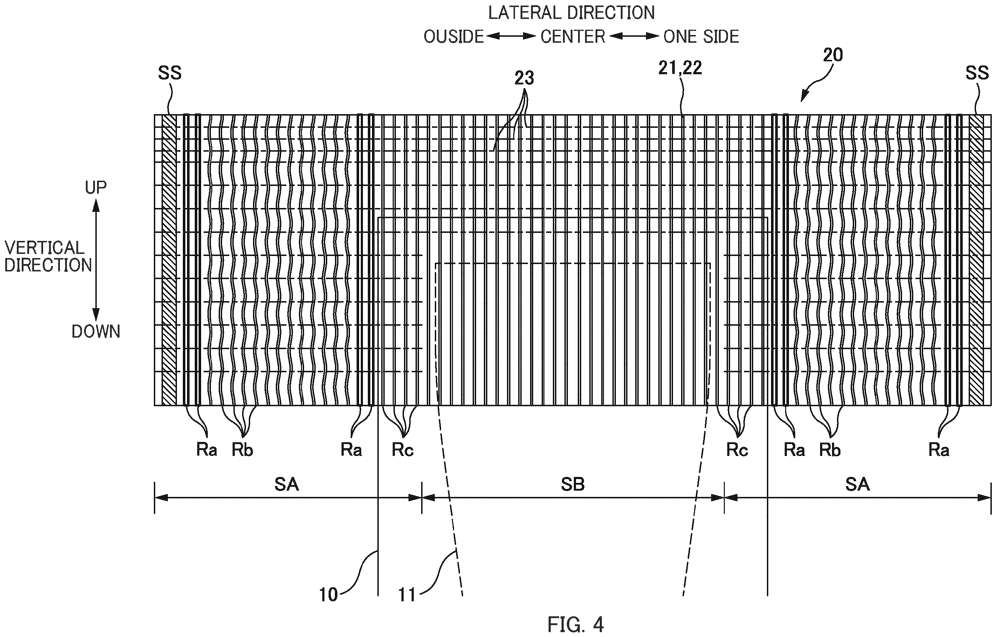

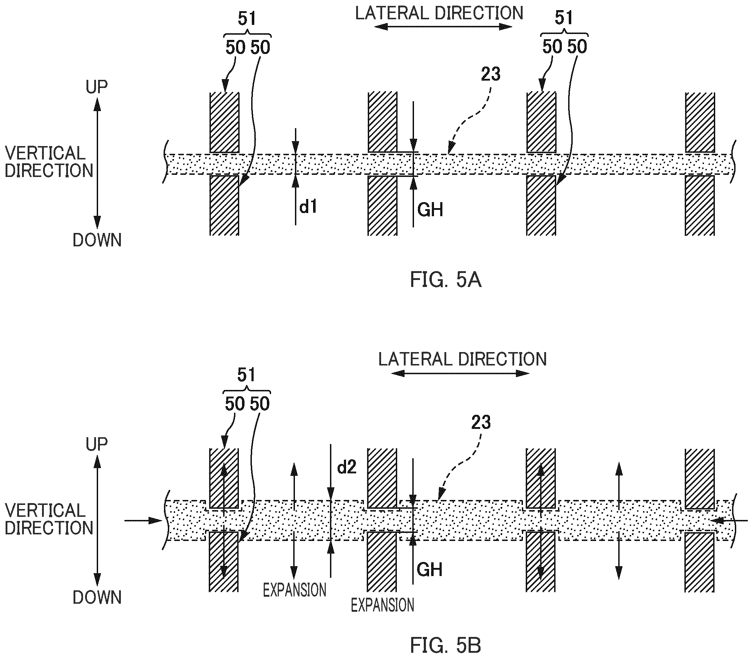

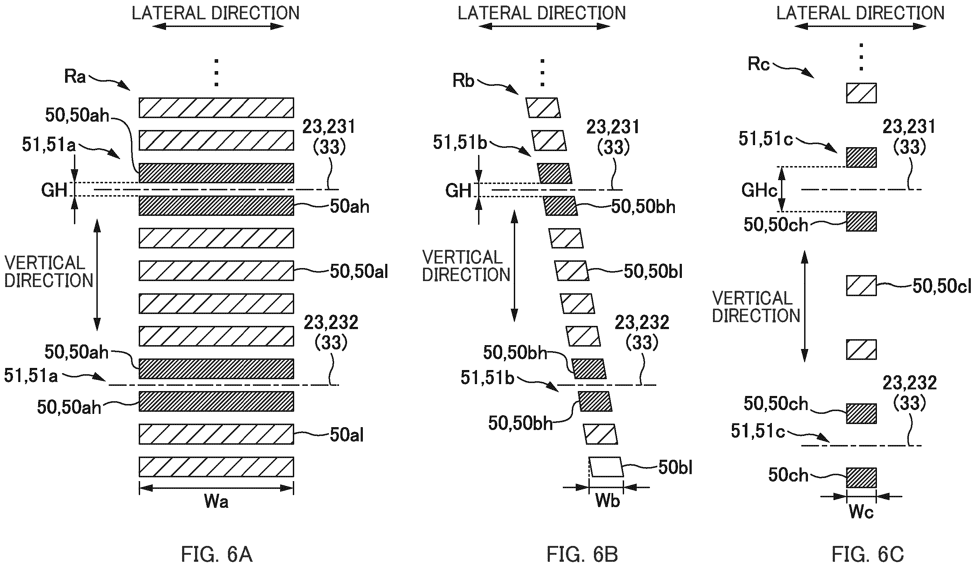

FIG. 4 is an illustrative diagram of weld portion rows Ra, Rb, and Rc provided in the front waist portion 20. FIGS. 5A and 5B are illustrative diagrams of attachment of the waist elastic members 23. FIGS. 6A to 6C are enlarged views of portions of the weld portion rows Ra, Rb, and Rc.

In FIG. 4 and the like, the front waist portion 20 is shown in a state of being stretched in the lateral direction. The state in which the waist portions 20 and 30 (stretchable regions) are stretched in the lateral direction is a state in which the waist portions 20 and 30 are stretched to the extent that wrinkles formed in the waist portions 20 and 30 are substantially no longer visible, that is to say a state of being stretched to the extent that the dimensions of constituent members of the waist portions 20 and 30 (e.g., the skin-side sheet 21) match or are close to the dimensions of the members on their own (i.e., the dimensions when the stretchability of the elastic members is not exhibited).

### Restriction of position of waist elastic members 23

The front waist portion 20 (back waist portion 30) is constituted by a stretchable sheet obtained by disposing the waist elastic members 23 (33) (elastic members), which can stretch and contract in the lateral direction, at intervals in the vertical direction between the skin-side sheet 21 (31) and the non-skin-side sheet 22 (32) (i.e., between a pair of sheets) that are joined to each other by weld portions 50. The skin-side sheet 21 (31) and the non-skin-side sheet 22 (32) are intermittently joined together by the weld portions 50 that are arranged discretely in the vertical direction and the lateral direction. The method for forming the weld portions 50 can be, for example, a well-known welding method such as ultrasonic welding or heat welding.

The pair of sheets joined by the weld portions 50 may be two separate sheets, such as the skin-side sheet 21 (31) and the non-skin-side sheet 22 (32) in the present embodiment, or may be two layers of a single sheet folded over onto itself. Furthermore, the weld portions 50 are not limited to joining only the pair of sheets sandwiching the waist elastic members 23 (33) therebetween, and may join three or more layers of sheets including a sheet overlaid on the pair of sheets.

Since the front waist portion 20 and the back waist portion 30 have roughly the same configuration, the front waist portion 20 will be described below as a representative of the two waist portions. The front waist portion 20 includes a plurality of weld portion rows Ra, Rb, and Rc in each of which weld portions 50 are arranged side by side in the vertical direction. The weld portion rows Ra, Rb, and Rc are arranged at intervals in the lateral direction. As shown in FIG. 5A, spaces (non-weld portions) through which the waist elastic members 23 pass are aligned in terms of vertical position, and the waist elastic members 23 can be arranged along the lateral direction. However, the positions of non-weld portions adjacent in the lateral direction may be offset from each other in the vertical direction, and even in such a case, the front waist portion 20 can be given stretchability in the lateral direction.

As shown in FIG. 5A, two weld portions 50 that are adjacent to each other in the vertical direction and sandwich a waist elastic member 23 therebetween will also be referred to as a "weld portion pair 51". The upper weld portion 50 and the lower weld portion 50 that make up the weld portion pair 51 are spaced apart by a gap GH in the vertical direction. The size of the gap GH is set the same as or slightly larger than an outer diameter d1 (GH ≥ d1) of the waist elastic member 23 in the state where the front waist portion 20 is fully stretched in the lateral direction.

However, the waist elastic member 23 becomes thicker as it contracts in the lateral direction. Therefore, as shown in FIG. 5B, while the waist elastic member 23 is contracted in the lateral direction (i.e., in the natural state of the front waist portion 20), the gap GH of the weld portion pair 51 is smaller than a maximum outer diameter d2 (GH < d2) of the waist elastic member 23. In this way, the laterally contracted waist elastic member 23 is sandwiched by the weld portion pair 51 (i.e., expansion in the vertical direction is restricted). Therefore, the position (movement), in the lateral direction and the vertical direction, of the waist elastic member 23 relative to the skin-side sheet 21 and the non-skin-side sheet 22 is restricted.

Therefore, even without using an adhesive to fix the waist elastic member 23 to the skin-side sheet 21 and the non-skin-side sheet 22, positional shifting of the waist elastic member 23 can be suppressed, and the stretchability of the front waist portion 20 can be maintained. In other words, by utilizing the weld portions 50, the waist elastic member 23 can be attached to the skin-side sheet 21 and the non-skin-side sheet 22 without using an adhesive or with a reduced amount of adhesive. Therefore, it is possible to suppress a decrease in the flexibility of the front waist portion 20 (stretchable sheet 40) caused by hardening of an adhesive. Also, it is possible to suppress deterioration of the elastic properties of the waist elastic member 23 caused by hardening of an adhesive.

Also, in the front waist portion 20, the waist elastic members 23 are fixed to the skin-side sheet 21 and the non-skin-side sheet 22 in the side joining portions SS. Specifically, during the manufacture of the diaper 1, the side joining portions SS are formed while the waist elastic members 23 are stretched and sandwiched between the skin-side sheet 21 and the non-skin-side sheet 22, and thus the end portions of the waist elastic members 23 are fixed to the side joining portions SS. Therefore, even if the front waist portion 20 is stretched a large amount when putting on the diaper 1, the waist elastic members 23 can be prevented from coming out from between the weld portion pairs 51, and the stretchability of the front waist portion 20 is maintained.

Furthermore, the relationship between the gap GH of the weld portion pair 51 and the outer diameters d1 and d2 of the waist elastic member 23 is not limited to the above description. For example, the waist elastic members 23 may partially overlap the weld portions 50 as long as the waist elastic member 23 is not cut. In this case as well, the laterally contracted waist elastic member 23 is sandwiched by the weld portion pair 51, and the position, in the lateral direction and the vertical direction, of the waist elastic member 23 relative to the skin-side sheet 21 and the non-skin-side sheet 22 is restricted.

Furthermore, there is no limitation to a configuration in which the positions of all of the waist elastic members 23 (elastic members) arranged in the front waist portion 20 are restricted by being sandwiched by the weld portion pairs 51. A configuration is possible in which the positions of some of the waist elastic members 23 are restricted by the weld portion pairs 51, and other waist elastic members 23 are fixed to the skin-side sheet 21 and the non-skin-side sheet 22 with an adhesive.

### Ease of stretching of waist portions 20 and 30

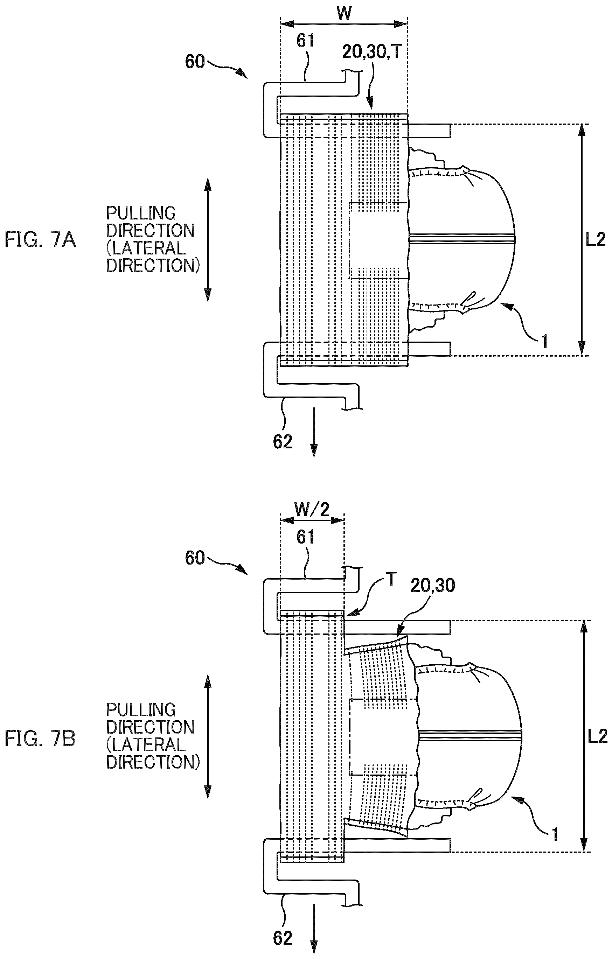

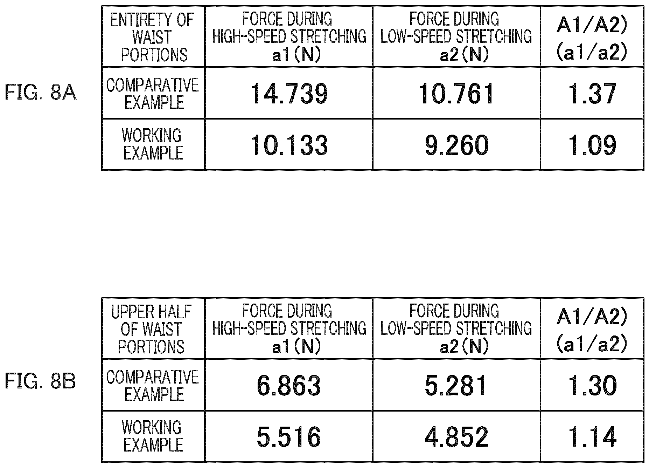

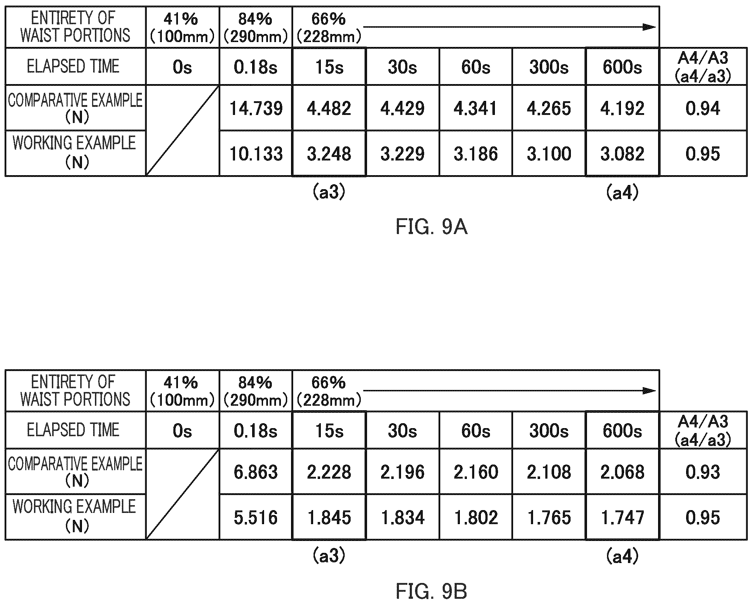

FIGS. 7A and 7B are illustrative diagrams of a tensile test performed on the waist portions 20 and 30. FIGS. 8A, 8B, 9A, and 9B show results of the tensile test.

In the diaper 1, the front waist portion 20 and the back waist portion 30 respectively have stretchable regions in which the waist elastic members 23 and 33 (elastic members), which can stretch and contract in the lateral direction, are arranged. When the user of the underpants-shaped diaper 1 (e.g., parent or caregiver) puts the diaper 1 on the wearer (child), the user first inserts their hands through the leg openings LH or the waist opening BH and quickly spreads the ring-like waist portions 20 and 30 in the lateral direction. Thereafter, the wearer's legs are passed through the waist opening BH and the leg openings LH, and the diaper 1 is pulled up to complete putting on the diaper 1.

At this time, the speed at which the user spreads the waist portions 20 and 30 in the lateral direction is fast, and the spreading can be completed in less than one second. On the other hand, while the diaper 1 is being worn, the waist portions 20 and 30 spread relatively slowly due to wearer movement such as breathing.

As a result of intensive research conducted by the applicant of the present invention, the following has been found regarding, for example, an ordinary waist portion in which the waist elastic members are fixed to the sheets by an adhesive such as a hot-melt adhesive applied in continuous lines in the longitudinal direction of the waist elastic members. It was found that when the waist portion was stretched at a high speed, the stretching stress (resistance of the waist portion during stretching) was higher than when the waist portion was stretched at a low speed, and the difference was large.

This is thought to be due to the influence of the adhesive that hardened while the waist portion was in the contracted state. When the waist portion is spread at a low speed, the hardened resin can deform gradually, but when the waist portion is spread at a high speed, the hardened resin needs to deform all at once, and it is thought that the resistance of the adhesive influences the stretching stress (resistance to spreading) of the waist portion. In particular, since diapers are packaged and distributed in the contracted state and remain in the contracted state for a long period of time, the hardness of the adhesive tends to increase. Therefore, it is thought that the waist portion of the diaper is more easily influenced by the hardening of the adhesive when the waist portion is first spread out after being taken out of the package in order to put the diaper on. If the user feels resistance to spreading of the waist portion when putting on the diaper 1 (a high amount of stress during high-speed stretching), the user may be given the impression that the product is tighter than the fit of the waist portion felt while actually being worn (a low amount of stress during low-speed stretching).

Therefore, in the diaper 1, the difference between the stretching stress when the waist portions 20 and 30 are stretched at a high speed and the stretching stress when the waist portions 20 and 30 are stretched at a low speed is made as small as possible. Specifically, the following applies. First, a part of the stretchable region of the waist portion 20 or 30 is used as a test piece T. Then, as shown in FIGS. 7A and 7B, the force (N) measured when the test piece T is stretched in the lateral direction to 84% of the maximum stretched length at a speed of 50000 mm/min is divided by the vertical width (mm) of the test piece T to obtain a first measurement value A1 (N/mm). Also, the force (N) measured when the test piece T is stretched in the lateral direction to 84% of the maximum stretched length at a speed of 500 mm/min is divided by the vertical width (mm) of the test piece to obtain a second measurement value A2 (N/mm). The value obtained when the first measurement value A1 is divided by the second measurement value A2 is smaller than 1.2. A1 (stress during high-speed stretching) / A2 (stress during low-speed stretching) < 1.2.

The stress during high-speed stretching (first measurement value A1) corresponds to the resistance that the user feels from the waist portions 20 and 30 when the user quickly spreads the waist portions 20 and 30 at the time of putting on the diaper 1. On the other hand, the stress during low-speed stretching (second measurement value A2) corresponds to the resistance (fit) felt by the wearer from the waist portions 20 and 30 during slow movement such as breathing.

Therefore, according to the diaper 1 of the present embodiment, the stress during high-speed stretching (first measurement value A1) is reduced, and the difference from the stress during low-speed stretching (second measurement value A2) is smaller than when the value obtained by dividing the first measurement value A1 by the second measurement value A2 is 1.2 or more. In other words, the difference between the ease of stretching of the waist portions 20 and 30 when putting on the diaper 1 and the ease of stretching of the waist portions 20 and 30 while the diaper 1 is being worn can be reduced. Therefore, when the waist portions 20 and 30 are quickly spread in order to put on the diaper 1, they can be spread with less force, thus making it easier to put on the diaper 1. It is also possible to suppress giving the user the impression that the product is tighter than the fit of the waist portions 20 and 30 felt while the diaper 1 is being worn (a low amount of stress during low-speed stretching).

In particular, in the case of the infant diaper 1 of the present embodiment or a nursing care diaper, the user (e.g., parent or caregiver) does not actually wear the diaper 1, and therefore the user is likely to judge the tightness felt during wearing based on how easily the waist portions 20 and 30 can be spread when putting on the diaper. Even in such a case, according to the diaper 1 of the present embodiment, the waist portions 20 and 30 can be easily spread even at a high speed, thus making it possible to suppress giving the user the impression that the product is very tight.

Furthermore, since the stress during high-speed stretching is suppressed, even when the wearer moves quickly, the waist portions 20 and 30 can stretch easily (have good conformability), and the wearer can move easily. Conversely, in the diaper 1, the stress during low-speed stretching is not too low, and therefore an appropriate fit during wearing can be obtained.

In the stretchable regions of the waist portions 20 and 30, the waist elastic members 23 and 33 are respectively disposed between a pair of sheets (21 and 22, 31 and 32) joined by the weld portions 50. While the waist elastic members 23 and 33 are contracted in the lateral direction, positions, in the vertical direction, of the waist elastic members 23 and 33 relative to the pairs of sheets are restricted by the waist elastic members 23 and 33 being sandwiched between pairs of weld portions 50 adjacent in the vertical direction, as shown in FIG. 5B.

Therefore, it is possible to reduce the amount of adhesive used to restrict the positions of the waist elastic members 23 and 33 relative to the sheets. Therefore, the influence of the hardening of the adhesive can be reduced even when the waist portions 20 and 30 are stretched at a high speed, and the stress during high-speed stretching (first measurement value A1) can be suppressed to reduce the difference from the stress during low-speed stretching (second measurement value A2).

However, the present invention is not limited to the above description, and the waist elastic members 23 and 33 may be attached to the corresponding sheets using an adhesive instead of the weld portions 50. In this case, in order to reduce the influence of adhesive hardening during high-speed stretching, it is sufficient to adjust, for example, the application pattern and the amount of adhesive such that the value obtained by dividing the first measurement value A1 by the second measurement value A2 is smaller than 1.2.

Also, in the present embodiment, the waist elastic members 23 and 33 are attached to the corresponding sheets by the weld portions 50 in both the front waist portion 20 and the back waist portion 30, but in one of the waist portions, the waist elastic members may be attached to the sheets with an adhesive. Also, in the case where the extension portion 30E is provided below the side joining portions SS as with the back waist portion 30, and elastic members are arranged along the lateral direction in the extension portion 30E as well (unlike FIG. 2), the elastic members may be attached to the sheet by the weld portions 50 or with an adhesive.

Also, the following is preferable in a test in which, as shown in FIGS. 7A and 7B, the test piece T is stretched to 84% of the maximum stretched length at a speed of 50000 mm/min and then, 15 seconds after the start of stretching, is allowed to contract to 66% of the maximum stretched length. First, the force (N) measured when the test piece T contracted to 66% of the maximum stretched length is divided by the vertical width (mm) of the test piece T to obtain a third measurement value A3 (N/mm) . Also, the force (N) measured 585 seconds after test piece T contracted to 66% of the maximum stretched length (i.e., 600 seconds after the start of the test) is divided by the vertical width (mm) of test piece T to obtain a fourth measurement value (N/mm). It is preferable that the value obtained by dividing the fourth measurement value A4 by the third measurement value A3 is 0.95 or more. It is preferable that A4 (stress when contraction stabilizes) / A3 (stress immediately after contraction) ≥ 0.95.

When putting on the diaper 1, the user spreads the waist portions 20 and 30 a large amount (e.g., to about 84% of the maximum stretched length), and then the waist portions 20 and 30 contract to fit to the wearer's waist (e.g., to about 66% of the maximum stretched length). Immediately after the waist portions 20 and 30 have contracted, a relatively large constricting force (stress when the waist portions 20 and 30 return to their original position, the third measurement value A3) acts on the wearer. This constricting force decreases over time and stabilizes.

According to the diaper 1 of the present embodiment, compared to when the value obtained by dividing the fourth measurement value A4 by the third measurement value A3 is smaller than 0.95, the third measurement value A3 immediately after contraction of the waist portions 20 and 30 is suppressed and approaches the fourth measurement value A4. This reduces the strong constricting force applied immediately after the waist portions 20 and 30 of the diaper 1 fit to the wearer, thereby reducing a sense of discomfort felt immediately after putting the diaper on. Conversely, even if the stretched state of the waist portions 20 and 30 continues during movement of the wearer, the fourth measurement value A4 does not decrease excessively, and it can be said that the fit is maintained. However, the present invention is not limited to the above description, and the value obtained by dividing the fourth measurement value A4 by the third measurement value A3 may be less than 0.95.

Also, the test piece T may be the entirety of the front waist portion 20 and the back waist portion 30 joined in a ring-like shape by the side joining portions SS, as shown in FIG. 7A. In this case, since the above relationship between the measurement values (A1/A2 < 1.2, A4/A3 ≥ 0.95) is established, it can be said that the difference in stress between when actually putting on the diaper 1 and while being in a state close to being worn is small, and the feeling of tightness immediately after putting on the diaper is reduced.

Furthermore, it is preferable that, even when the test piece T is a section Au (see FIG. 2) that is in the front waist portion 20 and the back waist portion 30 joined in a ring-like shape by the side joining portions SS and that is located above a vertical center CL of the side joining portion SS as shown in FIG. 7B, the above-mentioned relationship between the measurement values (A1/A2 < 1.2, A4/A3 ≥ 0.95) is satisfied.

The upper section Au of the waist portions 20 and 30 is more likely to influence the ease of spreading of the waist portions 20 and 30 and the feeling of tightness than the lower portion. Therefore, by suppressing the stress (A1) during high-speed stretching in the upper section Au of the waist portions 20 and 30 to reduce the difference from the stress (A2) during low-speed stretching, the waist portions 20 and 30 can be spread more easily, and the impression that the product is too tight can be reduced. Also, the feeling of tightness felt immediately after the waist portions 20 and 30 fit to the wearer can be reduced.

Furthermore, the test piece T is not limited to the waist portions 20 and 30 joined in a ring-like shape, and the test piece T may be the front waist portion 20 separated at the side joining portions SS, or the test piece T may be the back waist portion 30. In this case, it is preferable that the value obtained by dividing the first measurement value A1 by the second measurement value A2 when the back waist portion 30 is used as the test piece T is smaller than the value obtained by dividing the first measurement value A1 by the second measurement value when the front waist portion 20 is used as the test piece T. In other words, it is preferable that the stress during high-speed stretching (A1) of the back waist portion 30 is suppressed to reduce the difference from the stress during low-speed stretching (A2). This allows the back waist portion 30 to easily stretch even at a high speed, and allows the back waist portion 30 to be spread wide so as to be able to cover the buttocks when putting on the diaper 1.

Conversely, the value obtained by dividing the first measurement value A1 by the second measurement value A2 when the front waist portion 20 is used as the test piece T may be smaller than the value obtained by dividing the first measurement value A1 by the second measurement value A2 when the back waist portion 30 is used as the test piece T. In other words, the stress during high-speed stretching (A1) in the front waist portion 20 may be suppressed to reduce the difference from the stress during low-speed stretching (A2). In this case, the front waist portion 20 can be spread easily even at a high speed, and the front waist portion 20 can be spread easily when putting on the diaper 1, thereby making it possible to suppress the case where the wearer's feet (particularly the wearer's toes) get caught when passing the wearer's feet through the waist opening BH.

Furthermore, the absorbent article is not limited to the underpants-shaped diaper 1, and may be a tape-type diaper. In this case, for example, in the back waist portion of a tape-type diaper (the back region when the product length is divided into three), a stretchable region (so-called back gather portion) may be provided in the central portion in the lateral direction, or stretchable fastening tape (a stretchable region) may be provided on the left and right end portions (which protrude in the lateral direction from the main body portion). Even when these stretchable regions are used as the test piece T, the value obtained by dividing the first measurement value A1 by the second measurement value A2 is set smaller than 1.2. According to this configuration, the fastening tape and the back portion of the main body portion can stretch easily when putting on the tape-type diaper, and it is possible to suppress giving the user the impression that the product is too tight.

### Method of measuring first measurement value A1 and second measurement value A2

Next, a method for measuring the first measurement value A1 and the second measurement value A2 will be described. Measurement is carried out using a tensile tester (e.g., Autograph, Model AGS-1kNG from Shimadzu Corporation, or an equivalent thereof). The test environment is a room temperature of 23°C ±2°C and a humidity of 50% ±5%.
(1) First, two diapers 1 that have just been taken out of the package are prepared. In other words, two diapers 1 having test pieces T with the same configuration are prepared. For example, in FIG. 7A, the entirety of the front waist portion 20 and the back waist portion 30 joined in a ring-like shape is used as the test piece T. Therefore, the vertical width of the test piece T is the vertical length W of the side joining portions SS.

In FIG. 7B, the upper half region (Au) of the front waist portion 20 and the back waist portion 30 joined in a ring-like shape is used as the test piece T. Therefore, the vertical width of the test piece T is W/2, that is to say half the vertical length of the side joining portion SS. In this case, portions of the waist portions 20 and 30 are cut along cutout lines C1 shown in FIG. 2 so as not to influence the stretchability of the lower halves of the waist portions 20 and 30. The cutout lines C1 are lines that extend in the lateral direction from the left and right ends of the waist portions 20 and 30 to the side portions of the absorbent main body 10, at the vertical center CL of the waist portions 20 and 30.

As described above, the test piece T is a section that includes a part or the entirety of the stretchable regions of the waist portions 20 and 30. The test piece T is to be set in jigs 61 and 62 of the tensile tester 60, and the test is performed in a state where sections other than the test piece T (e.g., the absorbent main body 10 and the lower halves of the waist portions 20 and 30) remain connected to the test piece T. Therefore, elastic members other than the waist elastic members 23 and 33 that influence the stretchability in the lateral direction (e.g., leg elastic members (not shown) extending diagonally to the vertical direction along the leg openings LH) are cut in advance.

(2) Next, the distance between the jigs 61 and 62 of the tensile tester 60 is adjusted according to the size of the diaper 1. The distance from the upper end of the upper jig 61 to the lower end of the lower jig 62 is a jig separation length L2. In the present embodiment, the initial jig separation distance L2 (e.g., 140 mm) is set such that the waist portions 20 and 30 are stretched to approximately 41% (e.g., 38% to 45%) of the lateral maximum stretched length (e.g., L1 = 345 mm) of the waist portions 20 and 30.

(3) Next, the section of the diaper 1 that constitutes the test piece T is set between the jigs 61 and 62 of the tensile tester 60. The pulling direction of the tensile tester 60 is aligned with the stretch direction of the test piece T (the lateral direction of the diaper 1). When the test piece T is the waist portions 20 and 30 joined in a ring-like shape, the test piece T is set such that the side joining portions SS are located at the upper end of the upper jig 61 and the lower end of the lower jig 62. When the test piece T is not a ring, the test piece T (e.g., either the front waist portion 20 or the back waist portion 30, or the back waist portion of a tape-type diaper) is clamped with clip-type jigs, and the entirety of the absorbent article in an unfolded state is set in the tensile tester. In this case, the test piece T is set such that the center thereof in the direction orthogonal to the stretch direction (the vertical direction of the diaper 1) is aligned with the center in the jig width direction (direction orthogonal to the pulling direction).

(4) Next, the test piece T is stretched to 84% of the maximum stretched length (e.g., L2 = 345×0.84 = 290 mm) at a speed of 50000 mm/min, and the force (N) measured when stretched to 84% is obtained. The measured force is divided by the width (mm) of the test piece T to obtain the first measurement value A1 (N/mm).

Since the tensile tester 60 used in the present embodiment was not capable of handling a speed of 50000 mm/min, the jig 62 was pulled manually. Specifically, the jig was pulled a distance of 150 mm (= 290 mm - 140 mm) in approximately 0.18 seconds. During the manual stretching, the stretching speed is not limited to strictly 50000 mm/min, and a speed error of approximately ±10,000 mm/min is allowed. However, the measurement is not limited to manual pulling of the jig 62, and may be performed using a tensile tester that is compatible with high speeds.

(5) Next, the one test piece T (diaper 1) that has been stretched at a high speed is removed from the jigs 61 and 62, and the other test piece T (diaper 1) is set in the jigs 61 and 62 in a similar manner. The test piece T is stretched to 84% of the maximum stretched length (e.g., L2 = 290 mm) at a rate of 500 mm/min. The stretching is performed mechanically by the tensile tester 60, not manually. The force (N) measured when stretched to 84% is then obtained. The measured force is divided by the width (mm) of the test piece T to obtain the second measurement value A2 (N/mm).

(6) Lastly, the first measurement value A1 is divided by the second measurement value A2 (the result is rounded off to two decimal places), and it is determined whether the resulting value is smaller than 1.2. Also, since test pieces T that have the same width are stretched at different speeds, there is no need for conversion into force per unit width (N/mm). In other words, the value obtained when the force (N) obtained from the tensile tester 60 during high-speed stretching is divided by the force (N) obtained from the tensile tester 60 during low-speed stretching may be used as the value obtained when the first measurement value A1 is divided by the second measurement value A2.

### Method of measuring third measurement value A3 and fourth measurement value A4

Next, a method for measuring the third measurement value A3 and the fourth measurement value A4 will be described. (1) The test piece T is set in the jigs 61 and 62, and the test piece T is stretched to 84% of the maximum stretched length at a speed of 50000 mm/min, which is the same as the measurement method described above.

(2) Subsequently, 15 seconds after the start of the test, the test piece T is allowed to contract such that the length of the test piece T becomes 66% of the maximum stretched length (e.g., L2 = 228 mm = 345 × 0.66). The test piece T is allowed to contract at a high speed (50000 mm/min) similarly to when being stretched. If the tensile tester 60 is not capable of handling high speeds, the jig 62 is moved manually. Specifically, the jig moves 62 mm (= 290 - 228) in approximately 0.07 seconds, but when moved manually, there is no limitation to contracting strictly at a speed of 50000 mm/min. Then, the force (N) measured when the test piece T contracts to 66% of the maximum stretched length is obtained. The measured force is divided by the width (mm) of the test piece T to obtain the third measurement value A3 (N/mm).

(3) Then, the test piece T is kept in the state of being stretched to 66% of the maximum stretched length. The force (N) measured 600 seconds after the start of the test (i.e., 585 seconds after the test piece T has contracted in length from 84% to 66%) is obtained. The measured force is divided by the width (mm) of the test piece T to obtain the fourth measurement value A4 (N/mm).

(4) Then, the fourth measurement value A4 is divided by the third measurement value A3 (the result is rounded off to two decimal places), and it is determined whether the resulting value is 0.95 or more. Also, since the change pertains to measurement values of two test pieces T that have the same width, there is no need for conversion into force per unit width (N/mm). In other words, the value obtained when the force (N) obtained from the tensile tester 60 at 585 seconds after contraction is divided by the force (N) obtained from the tensile tester 60 immediately after contraction may be used as the value obtained when the fourth measurement value A4 is divided by the third measurement value A3.

### Working example

Diapers according to a comparative example and a working example were actually manufactured, and the above measurements were carried out. Both the diapers of the comparative example and the working example were underpants-shaped diapers having the configurations shown in FIGS. 1 to 3. The lateral maximum stretched length L1 of the waist portions 20 and 30 was 345 mm, and the vertical length W was 127 mm. The stretch factor of the waist elastic members 23 and 33 was 2.8 times, and the thickness was 470 dtex. Nine waist elastic members 23 were provided in the front waist portion 20, and ten waist elastic members 33 were provided in the back waist portion 30.

In the diaper of the comparative example, a hot-melt adhesive was applied in lines continuous in the longitudinal direction entirely around the waist elastic members 23 and 33, and the waist elastic members 23 and 33 were attached in a stretched state between the skin-side sheets 21 and 22 and the non-skin-side sheets 31 and 32.

In the diaper of the working example, as shown in FIGS. 4 to 6C, the waist elastic members 23 and 33 were attached between the skin-side sheets 21 and 22 and the non-skin-side sheets 31 and 32 using the weld portions 50. In the waist portions 20 and 30, the three types of weld portion rows Ra to Rc were formed at intervals of 5 mm in the lateral direction. As shown in FIG. 6A and the like, in the weld portion rows Ra to Rc, the weld portions 50 were continuous in the vertical direction in regions other than the regions where the waist elastic members 23 and 33 are located.

In the first weld portion rows Ra (FIG. 6A), weld portions 50 each having a lateral length Wa of 2.5 mm were arranged in a straight line in the vertical direction. The vertical length GH of the region provided with the waist elastic members 23 and 33 was 0.28 mm. As shown in FIG. 4, two first weld portion rows Ra were formed immediately inward of each of the side joining portions SS and immediately outward of the absorbent main body 10.

In the second weld portion rows Rb (FIG. 6B), weld portions 50 each having a lateral length Wb of 0.5 mm were arranged along the vertical direction so as to meander slightly in the lateral direction. The vertical length GH of the region provided with the waist elastic members 23 and 33 was 0.28 mm, likewise to the first weld portion rows Ra. The second weld portion rows Rb were formed outward of the absorbent main body 10 and between the first weld portion rows Ra.

In the third weld portion rows Rc (FIG. 6C), weld portions 50 each having a lateral length Wc of 0.5 mm were arranged in a straight line in the vertical direction. A vertical length GHc of the region provided with the waist elastic members 23 and 33 was wide at 0.6 mm. The third weld portion rows Rc were formed in regions overlapping the absorbent main body 10.

Note that in the weld portion rows Ra to Rc illustrated in FIGS. 6A to 6C, the weld portions 50 are arranged at intervals in the vertical direction. In the diaper produced as the working example, weld portions 50 that were continuous in the vertical direction were formed in the spaces other than the space where the waist elastic members 23 and 33 were located.

Values obtained by dividing the first measurement value A1 by the second measurement value A2 for the diapers of the comparative example and the working example are shown in FIGS. 8A and 8B. FIG. 8A shows results when the entirety of the waist portions 20 and 30 joined in a ring-like shape was used as the test piece T. In the diaper of the comparative example, the force (a1) measured during high-speed stretching was 14.739 (N), and the force (a2) measured during low-speed stretching was 10.761 (N). Therefore, the value obtained by dividing the first measurement value A1 by the second measurement value A2 (a1/a2) was 1.37, which was larger than 1.2.

On the other hand, in the diaper of the working example, the force (a1) measured during high-speed stretching was 10.133 (N), and the force (a2) measured during low-speed stretching was 9.260 (N). Therefore, the value obtained by dividing the first measurement value A1 by the second measurement value A2 (a1/a2) was 1.09, which was smaller than 1.2.

FIG. 8B shows the results when the upper half of the waist portions 20 and 30 joined in a ring-like shape was used as the test piece T. In the diaper of the comparative example, the force (a1) measured during high-speed stretching was 6.863 (N), and the force (a2) measured during low-speed stretching was 5.281 (N). Therefore, the value obtained by dividing the first measurement value A1 by the second measurement value A2 (a1/a2) was 1.30, which was larger than 1.2.

On the other hand, in the diaper of the working example, the force (a1) measured during high-speed stretching was 5.516 (N), and the force (a2) measured during low-speed stretching was 4.852 (N). Therefore, the value obtained by dividing the first measurement value A1 by the second measurement value A2 (a1/a2) was 1.14, which was smaller than 1.2.

From the above results, it was found that in the working example in which the waist elastic members 23 and 33 were attached using the weld portions 50, the stress during high-speed stretching (A1, a1) is suppressed, and the difference from the stress during low-speed stretching (A2, a2) can be reduced compared to the comparative example in which the waist elastic members 23 and 33 are attached using a large amount of adhesive. Therefore, the waist portions 20 and 30 can be easily spread quickly when putting on the diaper, and it is possible to suppress the impression that the product is too tight.

Furthermore, test subjects (21 people) were asked to evaluate how easily the diapers of the comparative example and the working example could be put on. 69% of the test subjects responded that the diaper of the working example had much better or somewhat better waist stretch than the diaper of the comparative example. 71% of the test subjects responded that the diaper of the working example was much easier or somewhat easier to put on compared to the diaper of the comparative example. Moreover, 69% of the test subjects responded that the diaper of the working example was not too constricting at all, or was somewhat less constricting than the diaper of the comparative example. From the above results, it was found that a diaper in which the value obtained by dividing the first measurement value A1 by the second measurement value A2 is smaller than 1.2 has good waist stretch, is easy to put on, and can give the impression of not being too tight.

FIGS. 9A and 9B show results obtained by dividing the fourth measurement value A4 by the third measurement value A3 for the diapers of the comparative example and the working example. FIG. 9A shows results when the entirety of the waist portions 20 and 30 joined in a ring-like shape was used as the test piece T. In the diaper of the comparative example, the measurement value immediately after contraction to 66% was 4.482 N, and the measurement value 600 seconds after the start of the test was 4.192 N. Therefore, the value obtained by dividing the fourth measurement value A4 by the third measurement value A3 (a4/a3) was 0.94, which was smaller than 0.95. On the other hand, in the case of the diaper of the working example, the measurement value immediately after contraction was 3.248 N, and the measurement value after 600 seconds was 3.082 N. Therefore, the value obtained by dividing the fourth measurement value A4 by the third measurement value A3 (a4/a3) was 0.95.

FIG. 9B shows results when the upper halves of the waist portions 20 and 30 were used as the test piece T. With the diaper 1 of the comparative example, the measurement value immediately after contraction was 2.228 N, the measurement value after 600 seconds was 2.068 N, and the value obtained by dividing the fourth measurement value A4 by the third measurement value A3 (a4/a3) was 0.93, which was smaller than 0.95. On the other hand, with the diaper of the working example, the measurement value immediately after contraction was 1.845 N, the measurement value after 600 seconds was 1.747 N, and the value obtained by dividing the fourth measurement value A4 by the third measurement value A3 (a4/a3) was 0.95.

From the above results, it was found that in the working example in which the waist elastic members 23 and 33 were attached by the weld portions 50, the stress (A3, a3) immediately after contraction to 66% was low, and the difference from the stress (A4, a4) when the contraction stabilized was also small. Therefore, with the diaper of the working example, it is possible to suppress causing the wearer to feel excessive tightness immediately after the waist portions 20 and 30 have been spread wide in order to put the diaper on and the waist portions 20 and 30 contract to fit the wearer. Also, there is little change in tightness (fit) while the diaper is being worn, and a sense of discomfort while wearing the diaper can be reduced.

### Other features

At least either the front waist portion 20 or the back waist portion 30 may be configured as follows. As shown in FIG. 4, in the case where a plurality of waist elastic members 23 (33) are arranged at intervals in the vertical direction in the waist portion 20 (30), it is preferable that all of the waist elastic members 23 (33) that are overlapped with the side joining portions SS with respect to the vertical direction are positionally restricted by the weld portions 50. In other words, it is preferable that for all of the waist elastic members 23 (33) provided in the waist portion 20 (30), while contracted in the lateral direction, positions, in the vertical direction, thereof relative to the pair of sheets (21 and 22, 31 and 32) are restricted by the waist elastic members 23 (33) being sandwiched between vertically adjacent two of the weld portions 50.

According to this configuration, the amount of adhesive applied to the waist portion 20 (30) can be further reduced, and the waist portion 20 (30) is not likely to be influenced by hardening of the adhesive even when stretched at a high speed. Therefore, the stress during high-speed stretching is suppressed, and the difference from the stress during low-speed stretching can be reduced. Therefore, the waist portion 20 (30) can be easily spread quickly, the diaper can be put on easily, and it is possible to suppress giving the user the impression that the product is too tight.

However, there is no limitation to the above description, and some of the waist elastic members 23 (33) (preferably four or fewer waist elastic members 23 (33)) of the waist portion 20 (30) may be attached to the sheets by an adhesive rather than by the weld portions 50.

As shown in FIG. 4, the waist elastic members 23 (33) may be positionally restricted by the weld portions 50 over the entire range in the lateral direction between the pair of side joining portions SS. In this case as well, the amount of adhesive applied to the waist portion 20 (30) can be reduced, the stress during high-speed stretching is suppressed, and the difference from the stress during low-speed stretching can be reduced. However, the present invention is not limited to the above description, and a configuration is possible in which one or more portions of the waist elastic members 23 in the lateral direction are positionally restricted by the weld portions 50, and the other portions are attached to the sheets by an adhesive.

When putting on the diaper 1, the left and right end portions of the waist portion 20 (30) is stretched and pulled up, for example. Therefore, the waist elastic members 23 (33) are more likely to tear and come off at the left and right end portions of the diaper 1 than at the central portion. Furthermore, in the case of the underpants-shaped diaper 1, the waist elastic members 23 (33) may be cut when the side joining portions SS are formed.

As shown in FIGS. 6A to 6C, the waist portion 20 (30) has a plurality of weld portion pairs 51 each including two weld portions 50 that are adjacent to each other in the vertical direction and sandwich a corresponding waist elastic member 23 (33). It is preferable that the weld portion pairs 51 include a weld portion pair in which the length GH in the vertical direction of the gap between the weld portions 50 is small (first weld portion pair), specifically weld portion pairs 51a in the first weld portion row Ra and weld portion pairs 51b in the second weld portion row Rb, and a weld portion pair in which the length GHc in the vertical direction of the gap between the weld portions 50 is large (second weld portion pair), located closer to the center in the lateral direction, specifically weld portion pairs 51c in the third weld portion row Rc. It is sufficient that the waist portion 20 (30) has at least one set of a first weld portion pair and a second weld portion pair. Furthermore, the second weld portion pair (the weld portion pair 51c in the third weld portion row Rc) does not need to be in contact with the waist elastic member 23 (33).

According to this configuration, in the left and right end portions of the waist portion 20 (30), the waist elastic members 23 (33) can be securely held by the weld portion pairs 51a in the first weld portion row Ra and the weld portion pairs 51b in the second weld portion row Rb. Therefore, even if the waist elastic members 23 (33) are cut at the side joining portions SS, the cut ends of the waist elastic members 23 (33) are likely to be held by the adjacent weld portions 51a and 51b. Therefore, stretchability of the waist portion 20 (30) is ensured over a wide range in the lateral direction.

On the other hand, in the central portion of the waist portion 20 (30) in the lateral direction, the vertical gap GHc between the weld portions 50 is large, thus making it possible to suppress cutting of the waist elastic members 23 (33) during formation of the weld portions 50. Even when the vertical gap GHc between the weld portions 50 is large, large shifting of the positions of the waist elastic members 23 (33) in the vertical direction can be restricted. Furthermore, the weld portions 50 can suppress rising of the skin-side sheet 21 (31) and the non-skin-side sheet 22 (32). Therefore, the amount of adhesive used in the waist portion 20 (30) can be reduced compared to when adhesive is used to prevent rising of the sheets. Therefore, the stress during high-speed stretching of the waist portion 20 (30) is suppressed, the difference from the stress during low-speed stretching can be reduced, and the waist portion 20 (30) can be easily spread quickly. Furthermore, in the central portion in the lateral direction (third weld portion rows Rc) of the waist portion 20 (30), the frictional force between the weld portions 50 and the waist elastic members 23 (33) is small, which can also be said to make it easier to spread the waist portion 20 (30).

Also, it is preferable that the weld portion pairs 51 include a weld portion pair in which the weld portions 50 have a long lateral length Wa (third weld portion pair), specifically the weld portion pairs 51a in the first weld portion row Ra, and weld portion pairs in which the weld portions 50 have a short lateral length Wb and Wc (fourth weld portion pairs), located closer to the center in the lateral direction, specifically the weld portion pairs 51b in the second weld portion row Rb and the weld portion pairs 51c in the third weld portion row Rc. It is sufficient that the waist portion 20 (30) has at least one set of a third weld portion pair and a fourth weld portion pair.

By increasing the length of the weld portions 50 in the lateral direction, the frictional force generated between the weld portions 50 and the waist elastic members 23 increases, and it is possible to suppress the case where the waist elastic members 23 (33) come out of the weld portion pairs 51. Therefore, even if the waist elastic members 23 (33) are cut at the side joining portions SS, the cut ends of the waist elastic members 23 (33) are likely to be held by weld portion pairs 51a in the vicinity thereof. Therefore, stretchability of the waist portion 20 (30) is ensured over a wide range in the lateral direction.

On the other hand, in regions distant from the side joining portions SS toward the center in the lateral direction, the waist elastic members 23 (33) do not need to be constrained more than necessary by the weld portion pairs 51. Therefore, by shortening the lengths Wb and Wc of the weld portions 50, the frictional force between the weld portions 50 and the waist elastic members 23 (33) is reduced. This makes it easier to spread the waist portion 20 (30), and makes it possible to suppress giving the impression that the product is too tight. Furthermore, shortening the lateral length of the stiffer weld portions 50 also ensures flexibility of the waist portion 20 (30).

Furthermore, as shown in FIG. 4, the waist elastic members 23 (33) overlapping the absorbent main body 10 may be cut so as to be discontinuous in the central portion of the diaper 1 in the lateral direction. In a region SB in which the waist elastic members 23 (33) are discontinuous portions, almost no stretchability is exhibited, and the stretching force of the waist elastic members 23 (33) per unit width in the vertical direction is lower than in a region SA in which the waist elastic members 23 (33) are continuous. Therefore, when putting on the diaper 1, for example, the waist portion 20 (30) can be spread easily. It is also possible to suppress contraction of the absorbent main body 10 caused by the waist elastic members 23 (33), thus ensuring flatness of the absorbent main body 10 (particularly the absorbent core 11) and improving close contact with the wearer.

Furthermore, when the waist elastic members 23 are cut during the process of manufacturing the diaper 1, the waist elastic members 23 contract and increase in diameter, and intermediate portions thereof are sandwiched between the weld portion pairs 51. Therefore, when the waist portion 20 (30) is stretched, the cut ends of the stretched waist elastic members 23 (33) are held by the weld portion pairs 51, thus ensuring stretchability in the continuous region SA.

In view of this, as shown in FIG. 4, in the regions (SA) where stretchability is desired, the first weld portion rows Ra including weld portions 50 having a long length in the lateral direction may be provided in the end portions toward the center in the lateral direction. According to this configuration, the cut ends of the waist elastic members 23, which were cut to form the discontinuous portions of the waist elastic members 23 (33), are securely held by the first weld portion rows Ra. Therefore, the stretchability of the waist portion 20 (30) (continuous portions SA) is ensured.

Also, as shown in FIGS. 6A to 6C, the weld portions 50 provided in the waist portion 20 (30) include first weld portions 50ah, 50bh, and 50ch that are adjacent to a certain waist elastic member 231 (first elastic member). The weld portions 50 also include second weld portions 50al, 50bl, and 50cl that are located in the central portion, in the vertical direction, between the waist elastic member 231 and a waist elastic member 232 (second elastic member) adjacent thereto in the vertical direction. In this case, the first weld portions 50ah, 50bh, and 50ch adjacent to the waist elastic member 23 (33) may have a stronger weld strength than the second weld portions 50al, 50bl, and 50cl.

According to this configuration, the waist elastic members 23 (33) can be securely held between the stiffer first weld portions 50ah and 50bh, and it is possible to suppress the case where the waist elastic members 23 (33) come out of the weld portion pairs 51. Moreover, positional shifting of the waist elastic members 23 (33) in the vertical direction can be reliably restricted by the stiffer first weld portions 50ch.

On the other hand, by lowering the weld strength of the second weld portions 50al, 50bl, and 50cl distant from the waist elastic members 23 (33), the flexibility of the waist portion 20 (30) can be ensured, and It is also possible to suppress rising of the skin-side sheet 21 (22) and the non-skin-side sheet 22 (32). Therefore, the amount of adhesive used in the waist portion 20 (30) can be reduced compared to when adhesive is used to prevent rising of the sheets. Therefore, the stress during high-speed stretching of the waist portion 20 (30) is suppressed, the difference from the stress during low-speed stretching can be reduced, and the waist portion 20 (30) can be easily spread quickly. The difference between the weld strength of the first weld portions 50ah, 50bh, and 50ch and the weld strength of the second weld portions 50al, 50bl, and 50cl can be visually determined based on the extent of melting of the sheets. Specifically, when the weld strength is strong, the sheets are completely turned into a film in the weld portion 50. When the weld strength is weak, fibers making up the nonwoven fabric remain in the weld portion.

In the present embodiment, the weld portions 50 are arranged at intervals in the vertical direction in the weld portion rows Ra to Rc. Therefore, the first weld portions 50ah, 50bh, and 50ch and the second weld portions 50al, 50bl, and 50cl are arranged discontinuously in the vertical direction. However, there is no limitation to the above description, and in regions other than where the waist elastic members 23 (33) are located, a configuration is possible in which the weld portions 50 is continuous in the vertical direction, and the first weld portions 50ah, 50bh, and 50ch and the second weld portions 50al, 50bl, and 50cl are continuous. Also, it is preferable that the number of second weld portions 50al, 50bl, and 50cl is greater than the number of first weld portions 50ah, 50bh, and 50ch, as shown in FIG. 6A and the like, or that the former are longer in the vertical direction than the latter (not shown). According to this configuration, the flexibility of the waist portion 20 (30) can be further ensured. For example, the weld portions 50 may be arranged at intervals in the vertical direction. Also, there is no limitation to the above description, and the first weld portions 50ah, 50bh, and 50ch and the second weld portions 50al, 50bl, and 50cl may have the same weld strength.

Furthermore, as shown in FIG. 5A, it is preferable that when the waist portion 20 (30) (stretchable region) is stretched in the lateral direction, the length GH in the vertical direction of the gap between two weld portions 50 that are adjacent in the vertical direction and sandwich a waist elastic member 23 (33) is larger than the length d1 in the vertical direction of the waist elastic member 23 (33) (GH>d1).

As the waist portion 20 (30) stretches in the lateral direction, the waist elastic members 23 (33) become thinner, and the vertical length also becomes shorter (from d2 to d1). If the waist elastic members 23 (33) are narrower than the gap GH between the weld portions 50 at that time, the frictional force generated between them can be reduced. This makes it easier to spread the waist portion 20 (30), and suppresses giving the user the impression that the product is too tight.

Although the weld portions 50 have been described above, the shape, number, arrangement, and the like of the weld portions 50 are not limited to the description given above. For example, in FIG. 4, the weld portion rows Ra to Rc are arranged at regular intervals in the lateral direction, but there is no limitation to this. For example, in the vicinity of the side joining portion SS where the waist elastic members 23 (33) may become torn, or at or in the vicinity of the side portions of the absorbent main body 10 where the waist elastic members 23 (33) are cut during manufacturing, the intervals between the weld portion rows Ra to Rc in the lateral direction may be narrower than in other regions. According to this configuration, the cut ends of the waist elastic members 23 (33) are securely gripped by the weld portion rows Ra to Rc.

As shown in FIG. 3, the underpants-shaped diaper 1 is provided with the absorbent main body 10 and an exterior body (front waist portion 20, back waist portion 30, and extension portion 30E) on the non-skin side thereof. It is preferable that when the side joining portion SS of the diaper 1 are separated and the diaper 1 is unfolded, the waist portion 20 (30) (stretchable regions) has a region 25 (35) in which the number of sheet layers constituting the exterior body is two layers, that is to say the exterior body is constituted by two sheets (skin-side sheet 21 (22) and non-skin-side sheet 22 (32)) sandwiching the waist elastic members 23 (33). In FIG. 3, the two-layer region 25 (35) is formed between the upper end of the absorbent main body 10 and the lower end of a folded-back portion 24 (34) of the non-skin-side sheet 22 (32).

By providing the region 25 (35) where the number of layers of sheets is smaller, it is possible to reduce the size of the region of the waist portion 20 (30) where sheets are stacked and joined with an adhesive, and it is possible to reduce the amount of adhesive used in the waist portion 20 (30). Therefore, the stress during high-speed stretching of the waist portion 20 (30) is suppressed, the difference from the stress during low-speed stretching can be reduced, and the waist portion 20 (30) can be easily spread quickly.

Also, in FIG. 3, at least one of the two sheets that sandwich the waist elastic members 23 (33) (here, the non-skin-side sheet 22 (32)) is provided with the folded-back portion 24 (35) in which the upper end portion of the sheet is folded downward in the vertical direction. In this case, it is preferable that a region with no adhesive is provided between the upper end of the absorbent main body 10 and the lower end of the folded-back portion 25 (35). In the present embodiment, the folded-back portion 24 (34) (non-skin-side sheet 22 (32)) is joined to the skin-side surface of the skin-side sheet 21 (31) with an adhesive, and the non-skin-side surface of the absorbent main body 10 is joined to the waist portion 20 (30) with an adhesive. The region 25 (35) therebetween is a region with no adhesive.

In this way, when a region with no adhesive is provided in the waist portion 20 (30), the amount of adhesive used in the waist portion 20 (30) can be reduced. Therefore, the stress during high-speed stretching of the waist portion 20 (30) is suppressed, the difference from the stress during low-speed stretching can be reduced, and the waist portion 20 (30) can be easily spread quickly.

The region with no adhesive may also be a partial region with no adhesive in a region where adhesive is applied in a known pattern (e.g., a spiral pattern or an Ω-shaped pattern). In other words, the region with no adhesive may be a region other than a region completely covered with an adhesive (a so-called solidly coated region).

Furthermore, as shown in FIG. 2, it is preferable that, when the diaper 1 is in an unfolded state, in a section 11b of the absorbent core 11 overlapped with the back waist portion 30 in the thickness direction thereof, the average basis weight (g/m²) of the absorbent core 11 is lower than in a section 11f of the absorbent core 11 overlapped with the front waist portion 20 in the thickness direction thereof.

In this case, the stiffness of the portion of the absorbent core 11 located in the back waist portion 30 is reduced, and thus the back waist portion 30 can stretch more easily. Therefore, when putting on the diaper 1, the back waist portion 30 can be spread sufficiently to cover the buttocks. On the other hand, the basis weight of the absorbent core 11 is higher in the front waist portion 20 close to the urination area, thus making it possible to suppress leakage of excreted fluid.

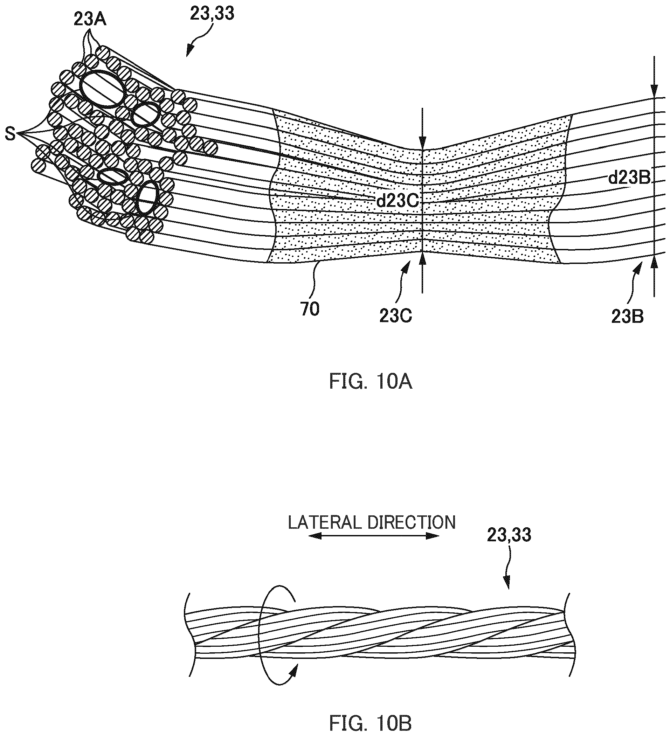

FIGS. 10A and 10B are illustrative diagrams of the waist elastic member 23 (33). It is preferable that a lubricant is applied to at least either the surface of the waist elastic member 23 (33) or the inward surfaces of the pair of sheets 21 and 22 (31 and 32) that sandwich the waist elastic member 23 (33). In FIG. 10A, a lubricant 70 is applied to the waist elastic member 23 (33). Although not shown, a lubricant may be applied to the non-skin-side surface of the skin-side sheet 21 (31) and the skin-side surface of the non-skin-side sheet 22 (32).

According to this configuration, it is possible to reduce the frictional force between the waist elastic members 23 (33) and the skin-side sheet 21 (22) and the non-skin-side sheet 31 (32). This makes it easier to spread the waist portion 20 (30), and suppress giving the user the impression that the product is too tight.

Furthermore, in the manufacturing process of the diaper 1, even if the waist elastic members 23 (33) are pressed by the protrusion pattern for forming the weld portions 50 and the side joining portions SS, the waist elastic members 23 (33) can slide easily due to the lubricant 70. Therefore, the waist elastic members 23 (33) are easily pushed out from the protrusion pattern, thus making it possible to suppress cutting of the waist elastic members 23 (33).

One example of the lubricant is an oil. The type of oil is not particularly limited, and may be, for example, an oil applied to elastic fibers 23A constituting the waist elastic members 23 (33) in order to prevent adhesion, improve processability, or the like. Specific examples of applicable oils include oils containing dimethylpolysiloxane, modified polysiloxanes in which some of the methyl groups have been replaced with other alkyl groups, phenyl groups, amino groups, or the like, and oils having mineral oil, as their main component.

As shown in FIG. 10A, it is preferable that the waist elastic member 23 (33) is a string-like elastic member formed by assembling a plurality of elastic fibers 23A. According to this configuration, even if some of the elastic fibers 23A are cut during the process of forming the side joining portions SS, the remaining elastic fibers 23A remain connected, thus preventing cutting of the waist elastic member 23 (33). Therefore, it is possible to suppress the case where the waist elastic member 23 (33) comes out from the weld portion pair 51, and the stretchability of the waist portion 20 (30) can be ensured.

Also, with the configuration including a plurality of elastic fibers 23A, voids S are likely to be formed between the elastic fibers 23A. Therefore, even if the waist elastic members 23 (33) are compressed by the protrusion pattern for forming the side joining portions SS and the weld portions 50, the voids S become compressed to create escape spaces (i.e., the elastic members are not squashed by the protrusion pattern), thereby suppressing cutting of the waist elastic members 23 (33).

Furthermore, with the configuration including a plurality of elastic fibers 23A, the thickness of the waist elastic member 23 (33) tends to vary in portions depending on how the elastic fibers 23A are gathered. For example, as shown in FIG. 10A, the waist elastic member 23 (33) has a large diameter portion 23B in which the outer diameter is a first outer diameter d23B, and a small diameter portion 23C (groove portion) having an outer diameter d23C smaller than the first outer diameter d23B. According to this configuration, when the large diameter portion (23B) of the elastic member 23 (33) is positioned between a weld portion pair 51, the large diameter portion is tightly sandwiched by the weld portion pair 51, thereby suppressing the case where the waist elastic member 23 (33) comes out of the weld portion pair 51.

Moreover, it is preferable that the waist elastic members 23 (33) are disposed in a twisted state between the pair of sheets 21 and 22 (31 and 31). This increases the surface area of the waist elastic members 23 (33), increases the frictional force with the sheets (facilitates entanglement with the sheet fibers), and makes it possible to suppress the case where the waist elastic members 23 come out of the weld portion pairs 50. Therefore, the stretchability of the waist portion 20 (30) is ensured.

Moreover, it is preferable that the waist elastic members 23 (33) are transparent and do not contain pigments (e.g., white titanium oxide). According to this configuration, the surface of the waist elastic members 23 is smooth, and it is possible to reduce the frictional force between the waist elastic members 23 (33) and the skin-side sheet 21 (31) and non-skin-side sheet 22 (32). Therefore, the waist portion 20 (30) can be spread easily.

The above-described embodiments are intended to facilitate understanding of the present invention, and are not intended to limit the present invention. The present invention can be modified or improved without departing from the gist of the invention, and it goes without saying that the present invention includes equivalents thereof.

### REFERENCE SIGNS LIST

1 diaper (absorbent article),
10 absorbent main body, 11 absorbent core, 12 top sheet,
13 back sheet, 14 exterior sheet, 15 leg elastic member,
16 leak-proof-wall elastic member,
20 front waist portion,
21 skin-side sheet (sheet), 22 non-skin-side sheet (sheet),
23 waist elastic member (elastic member, first elastic member),
23A elastic fiber, 24 folded-back portion,
30 back waist portion, 30E extension portion,
31 skin-side sheet (sheet), 32 non-skin-side sheet (sheet),
33 waist elastic member (elastic member), 34 folded-back portion,
SS side joining portion,
50 weld portion,
60 tensile tester, 61 jig, 62 jig,
70 lubricant
T test piece

## Claims

1. An absorbent article having a vertical direction and a lateral direction that intersect each other,
the absorbent article comprising:
an absorbent core;
a front waist portion; and
a back waist portion,
at least either of the front waist portion and the back waist portion having a stretchable region in which an elastic member capable of stretching and contracting in the lateral direction is arranged, and
in a test performed on a test piece that is at least a portion of the stretchable region, a value obtained by dividing a first measurement value by a second measurement value being smaller than 1.2,
the first measurement value being a value obtained when a force measured when the test piece is stretched in the lateral direction to 84% of a maximum stretched length at a speed of 50000 mm/min is divided by a width of the test piece in the vertical direction, and
the second measurement value being a value obtained when a force measured when the test piece is stretched in the lateral direction to 84% of the maximum stretched length at a speed of 500 mm/min is divided by the width of the test piece.

2. The absorbent article according to claim 1, wherein
in a test in which the test piece is stretched to 84% of the maximum stretched length at a speed of 50000 mm/min and then, 15 seconds after a start of stretching, is allowed to contract to 66% of the maximum stretched length,
a value obtained by dividing a fourth measurement value by a third measurement value is 0.95 or more,
the third measurement value being a value obtained when a force measured when the test piece contracted to 66% of the maximum stretched length is divided by the width, and
the fourth measurement value being a value obtained when a force measured 585 seconds after the test piece contracted to 66% of the maximum stretched length is divided by the width.

3. The absorbent article according to claim 1 or 2, wherein
the absorbent article is an underpants-shape absorbent article in which the front waist portion and the back waist portion are joined to each other by a pair of side joining portions at two lateral side portions, and
the test piece is a section that is in the front waist portion and the back waist portion joined in a ring-like shape by the side joining portions and that is located above a center, in the vertical direction, of the side joining portions.

4. The absorbent article according to claim 1 or 2, wherein
in the stretchable region, the elastic member is arranged between a pair of sheets joined together by a plurality of weld portions, and
while the elastic member is contracted in the lateral direction, a position, in the vertical direction, of the elastic member relative to the pair of sheets is restricted by the elastic member being sandwiched between two of the weld portions adjacent in the vertical direction.

5. The absorbent article according to claim 4, wherein
the absorbent article is an underpants-shape absorbent article in which the front waist portion and the back waist portion are joined to each other by a pair of side joining portions at two lateral side portions,
in at least either of the front waist portion and the back waist portion,
a plurality of the elastic members are arranged at intervals in the vertical direction, and
for all of the elastic members that are overlapped with the side joining portions with respect to the vertical direction, while contracted in the lateral direction, positions, in the vertical direction, of the overlapping elastic members relative to the pair of sheets are restricted by the overlapping elastic members being sandwiched between vertically adjacent two of the weld portions.

6. The absorbent article according to claim 4, wherein
the plurality of weld portions include a plurality of weld portion pairs each including two weld portions that are adjacent in the vertical direction and sandwich a corresponding elastic member,
the plurality of weld portion pairs include a first weld portion pair, and a second weld portion pair located closer to a center in the lateral direction than the first weld portion pair is, and
in the second weld portion pair, a distance in the vertical direction between the weld portions sandwiching the corresponding elastic member is larger than in the first weld portion pair.

7. The absorbent article according to claim 4, wherein
the plurality of weld portions include a plurality of weld portion pairs each including two weld portions that are adjacent in the vertical direction and sandwich a corresponding elastic member,
the plurality of weld portion pairs include a third weld portion pair, and a fourth weld portion pair located closer to a center in the lateral direction than the third weld portion pair is, and
in the fourth weld portion pair, lengths of the weld portions in the lateral direction are smaller than in the third weld portion pair.

8. The absorbent article according to claim 4, wherein
the stretchable region includes a first elastic member and a second elastic member adjacent to each other with an interval therebetween in the vertical direction,
the plurality of weld portions include a first weld portion adjacent to the first elastic member, and a second weld portion located in a central portion, in the vertical direction, between the first elastic member and the second elastic member, and
the first weld portion has a higher weld strength than the second weld portion.

9. The absorbent article according to claim 4, wherein
while the stretchable region is stretched in the lateral direction,
a length in the vertical direction of a gap between the two weld portions that are adjacent in the vertical direction and sandwich the elastic member is greater than a length in the vertical direction of the elastic member.

10. The absorbent article according to claim 1 or 2, wherein
the absorbent article is an underpants-shape absorbent article in which the front waist portion and the back waist portion are joined to each other by a pair of side joining portions at two lateral side portions,
the absorbent article further comprises:
an absorbent main body including the absorbent core; and
an exterior body, and
while the absorbent article is in an unfolded state, the stretchable region has a region in which a number of sheet layers constituting the exterior body is two layers corresponding to a pair of sheets sandwiching the elastic member.

11. The absorbent article according to claim 1 or 2, wherein
the absorbent article is an underpants-shape absorbent article in which the front waist portion and the back waist portion are joined to each other by a pair of side joining portions at two lateral side portions,
the absorbent article further comprises an absorbent main body including the absorbent core, and
at least either of the front waist portion and the back waist portion includes
a folded-back portion in which an upper end portion of at least either of a pair of sheets sandwiching the elastic member is folded downward in the vertical direction, and
a region with no adhesive, between an upper end of the absorbent main body and a lower end of the folded-back portion.

12. The absorbent article according to claim 1 or 2, wherein
a lubricant is applied to at least either of a surface of the elastic member and inward surfaces of the pair of sheets sandwiching the elastic member.

13. The absorbent article according to claim 1 or 2, wherein
the value obtained by dividing the first measurement value by the second measurement value when the back waist portion is the test piece is smaller than the value obtained by dividing the first measurement value by the second measurement value when the front waist portion is the test piece.

14. The absorbent article according to claim 1 or 2, wherein
the value obtained by dividing the first measurement value by the second measurement value when the front waist portion is the test piece is smaller than the value obtained by dividing the first measurement value by the second measurement value when the back waist portion is the test piece.

15. The absorbent article according to claim 1 or 2, wherein
the absorbent article is an underpants-shape absorbent article in which the front waist portion and the back waist portion are joined to each other by a pair of side joining portions at two lateral side portions,
while the absorbent article is in an unfolded state, in a section of the absorbent core overlapped with the back waist portion in a thickness direction of the back waist portion, an average basis weight of the absorbent core is lower than in a section of the absorbent core overlapped with the front waist portion in a thickness direction of the front waist portion.
